# FASCICULE DE BREVET EUROPEEN

(11) **EP 0 716 858 B1**
(45) Date de publication et mention de la délivrance du brevet: **14.06.2000**
(21) Numéro de dépôt: 95440068.5
(22) Date de dépôt: 25.10.1995
(51) Int. Cl.: A61L 9/12

(54) **Dispositif diffuseur de parfum à diffusion réglable**
Duftstoffspender mit einstellbarer Abgabe
Perfume dispenser with adjustable diffusion

(30) Priorité: 26.10.1994 FR 9413021
(43) Date de publication de la demande: 19.06.1996
(73) Titulaire: Scheuer, Jean-Louis, 67320 Drulingen (FR); Felten, Marc, 6700 Strasbourg (FR)
(72) Inventeur: Scheuer, Jean-Louis, 67320 Drulingen (FR); Felten, Marc, 6700 Strasbourg (FR)
(74) Mandataire: Rhein, Alain

(56) Documents cités:
- FR-A- 1 500 142
- US-A- 1 782 919
- US-A- 2 738 225

## Description

La présente invention a pour objet un dispositif diffuseur de parfum désodorisant à diffusion réglable.

On connaît déjà de nombreux types de diffuseurs de parfum, qui se présentent généralement sous la forme d'une boîte de matière plastique contenant un produit parfumé, et dans laquelle sont pratiquées des ouvertures permettant la diffusion du parfum.

Ces diffuseurs, s'ils sont de conception simple, présentent néanmoins l'inconvénient d'être d'un coût de fabrication relativement élevé pour un objet jetable non réutilisable.

On connaît également des dispositifs diffuseurs qui consistent en une simple plaquette d'un matériau absorbant, imprégné d'un produit parfumé, mais ce type de diffuseur ne permet pas de réguler la diffusion du parfum.

Le document FR-A- 1 500 142 décrit un appareil odorisant ou désodorisant, notamment pour véhicule, qui peut être constitué par au moins deux tubes concentriques dont celui extérieur est posé ou même fixé dans le véhicule et celui intérieur renferme une substance odorisante ou désodorisante, ces tubes comportant chacun, également réparties sur leur périphérie, des découpes de forme quelconque, aptes à être mises en regard les unes avec les autres pour assurer la mise en service de l'appareil.

Le document décrit en détail une mise en coïncidence des découpes par rotation mais indique qu'il est également possible d'envisager une mise en oeuvre par translation ou translation et rotation. Les découpes peuvent alors être présentes sur le seul tube intérieur.

La présente invention propose un diffuseur de parfum désodorisant à régulation de diffusion différent, d'un coût de fabrication faible tout en étant d'une efficacité similaire à celle des diffuseurs à régulation existants, et d'une manipulation simple puisque nécessitant un simple mouvement de translation.

Le dispositif diffuseur de parfum désodorisant selon l'invention comporte un étui en matériau semi-rigide muni d'un certain nombre d'orifices, dans lequel est introduit un second étui comportant un même nombre d'orifices susceptibles d'être mis en regard des orifices de l'étui extérieur par coulissement dans celui-ci de l'étui intérieur, et il se caractérise essentiellement en ce que les deux étuis sont sensiblement plats et en ce que les orifices sont ménagés en bordure desdits étuis, à cheval sur leurs plis longitudinaux, l'étui intérieur renfermant une plaquette d'un matériau imprégné d'un produit parfumé.

Selon une caractéristique additionnelle du dispositif selon l'invention un orifice est percé dans l'étui extérieur, à proximité du bord supérieur de celui-ci, en vue de l'insertion d'un organe de suspension.

Selon une autre caractéristique additionnelle du dispositif selon l'invention l'étui intérieur comporte à proximité de son bord supérieur un orifice de forme oblongue dans le sens longitudinal, permettant à l'orifice de l'étui extérieur d'être en permanence en regard dudit orifice oblong lors du coulissement de l'étui intérieur dans l'étui extérieur.

Les étuis du dispositif diffuseur selon l'invention sont préférentiellement, mais non limitativement, réalisés en carton ou en matière plastique, et peuvent être munis d'une impression, par exemple publicitaire, sur au moins une des faces de l'étui extérieur.

Le dispositif selon l'invention peut être suspendu par un organe de suspension, par exemple au rétroviseur intérieur d'un véhicule automobile, la régulation de la diffusion du parfum désodorisant étant obtenue en faisant coulisser l'étui intérieur dans l'étui extérieur afin de mettre en regard complètement ou partiellement les orifices de l'un avec les orifices de l'autre, opération facile qui peut se faire même en conduisant le véhicule.

Le dispositif diffuseur peut également, si sa suspension n'est pas réalisable, être posé sur une surface plane, ou bien dressé verticalement en insérant sa base dans une fente ou entre deux objets, ou encore collé au moyen d'une pastille adhésive.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue en élévation en éclaté d'un dispositif selon l'invention.
- la figure 2 représente une vue en développé d'une partie du même dispositif.
- la figure 3 représente une vue en développé d'une autre partie du même dispositif.

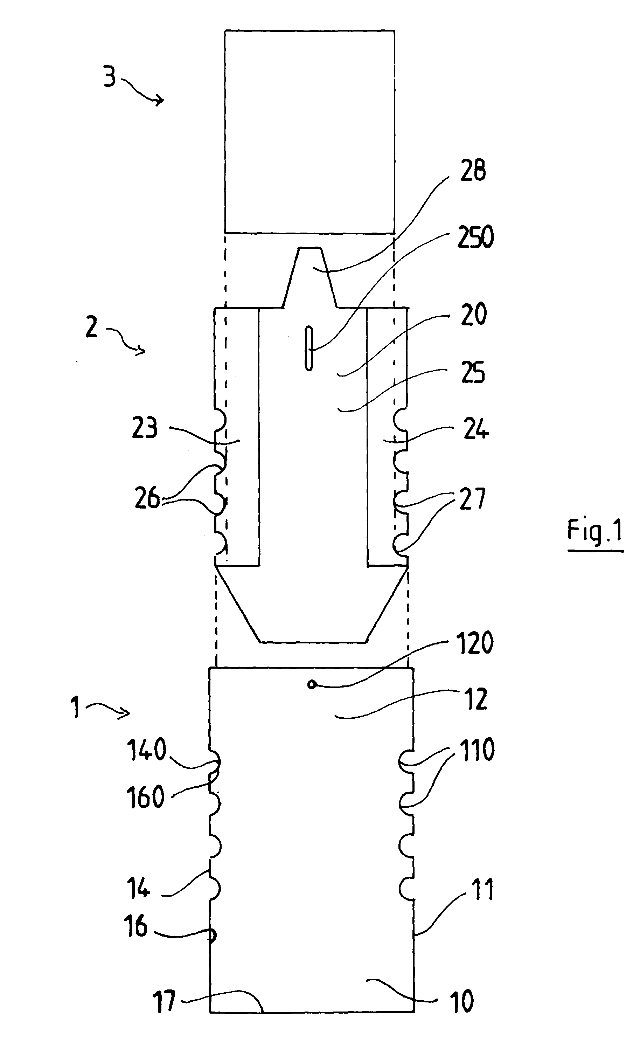

Si on se réfère à la figure 1 on peut voir qu'un dispositif diffuseur selon l'invention comporte un étui extérieur 1 dans lequel peut coulisser un étui intérieur 2, dans lequel est introduite une plaquette 3 imprégnée d'un produit parfumé.

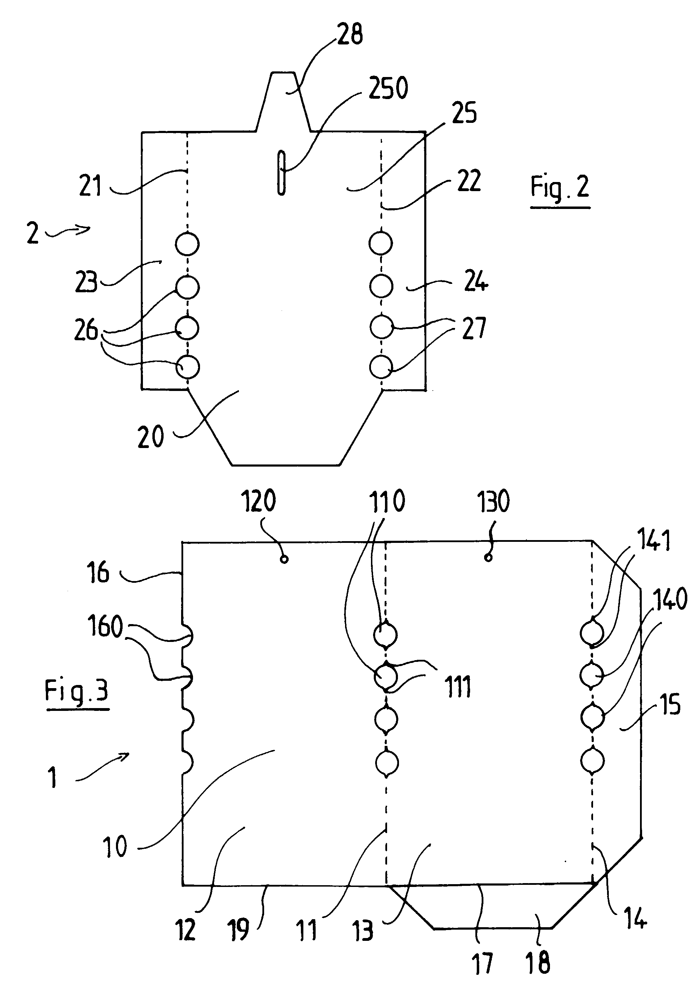

Si on se réfère également à la figure 2 on peut voir que l'étui intérieur 2 est essentiellement constitué d'une feuille 20 comportant latéralement deux plis longitudinaux 21 et 22 permettant de rabattre les parties latérales 23 et 24 sur la partie centrale 25, afin de maintenir la plaquette 3, le maintien pouvant éventuellement être complété par collage ou agrafage.

Des orifices 26 sont pratiqués dans la partie centrale 25 et la partie latérale 23, à cheval sur le pli 21, et de même, des orifices 27 sont pratiqués dans la partie centrale 25 et la partie latérale 24, à cheval sur le pli 22.

Les produits parfumés étant généralement gras, il est préférable que la plaquette 3 ne soit pas en contact avec les parois de l'étui extérieur 1, lesquelles pourraient absorber ledit produit, notamment si l'étui extérieur 1 est réalisé en carton. Il convient alors d'isoler la plaquette 3, par exemple en faisant des rabats latéraux 23 et 24 de grande dimension, ou en adjoignant un troisième rabat, non représenté, solidaire du bord inférieur de la partie centrale, sur lequel la plaquette 3 peut être collée ou agrafée.

Si on se réfère également à la figure 3 on peut voir que l'étui extérieur 1 est essentiellement constitué d'une feuille 10 comportant d'une part un pli longitudinal 11 sensiblement médian de manière à constituer deux faces 12 et 13, d'autre part un pli longitudinal 14 le long de la face 13, délimitant une languette longitudinale 15 de solidarisation par collage au dos de la face 12, le long de son bord libre 16, et d'autre part encore un pli 17 transversal le long de la base de la face 13, délimitant une languette transversale 18 de solidarisation par collage au dos de la face 12, le long de son bord inférieur 19.

Des orifices ronds 110 sont pratiqués dans les faces 12 et 13 à cheval sur le pli 11, et des orifices ronds 140 sont pratiqués dans la face 13 et la languette 15 à cheval sur le pli 14, alors que des orifices semi-circulaires 160 sont pratiqués sur le bord libre 16 de la face 12.

Après pliage et collage de la feuille 10, les faces 12 et 13 sont dos à dos et les orifices semi-circulaires 160 sont en regard des orifices ronds 140.

Lorsque l'étui intérieur 2 est placé dans l'étui extérieur 1, par coulissement de l'étui intérieur 2 dans l'étui extérieur 1, on peut mettre plus ou moins en regard les orifices 26 et 27 de l'étui intérieur 2 avec respectivement les orifices 140 et 110, laissant ainsi passer plus ou moins le parfum.

Il convient de remarquer que les orifices 110 et 140 ne sont pas parfaitement ronds, mais qu'ils comportent des échancrures 111 et 141 au niveau des plis 11 et 14, afin de supprimer les angles vifs susceptibles de créer des points d'accrochage lors de l'introduction de l'étui intérieur 2 dans l'étui extérieur 1.

L'étui intérieur 2 comporte à sa partie supérieure une languette 28 facilitant sa préhension en vue de réaliser le coulissement.

On peut voir également sur ces figures que l'étui intérieur 2 comporte dans la partie supérieure de sa partie centrale 25 un orifice 250 oblong dans le sens longitudinal, et que l'étui extérieur 1 comporte dans la partie supérieure de ses faces 12 et 13 des orifices 120 et 130 qui sont mis en regard lors du pliage, et qui se trouvent en regard de l'orifice 250 lorsque l'étui intérieur 2 est introduit dans l'étui extérieur 1.

Les orifices 120, 250 et 130 sont destinés à être traversés par un organe de suspension, non représenté, la forme oblongue de l'orifice 250 autorisant le coulissement de l'étui intérieur 2 dans l'étui extérieur 1.

L'organe de suspension peut être par exemple un fil de matière plastique dont une partie est formée en boucle et dont une extrémité comporte un moyen de fixation, du type harpon encliquetable dans les orifices 120, 250 et 130.

## Revendications

1. Dispositif diffuseur de parfum désodorisant à diffusion réglable comprenant un premier étui extérieur (1) en matériau semi-rigide, comportant un certain nombre d'orifices (110, 140), dans lequel est introduit un second étui intérieur (2) muni du même nombre d'orifices (26, 27) susceptibles d'être placés en regard des orifices (140, 110) de l'étui extérieur (1) par coulissement dans ledit étui extérieur (1) de l'étui intérieur (2), caractérisé en ce que les deux étuis (1, 2) sont sensiblement plats et en ce que les orifices (110, 140 ; 26, 27) sont ménagés en bordure desdits étuis, à cheval sur leurs plis longitudinaux (11,14; 21, 22), l'étui intérieur (2) renfermant une plaquette (3) d'un matériau imprégné d'un produit parfumé.

2. Dispositif selon la revendication 1 caractérisé en ce que les orifices (110, 140) pratiqués dans l'étui extérieur (1) sont ronds avec une échancrure (111, 141) au niveau du pli (11, 14).

3. Dispositif selon la revendication 1 ou la revendication 2 caractérisé en ce que l'étui intérieur (2) et l'étui extérieur (1) sont en carton ou en matière plastique, l'étui intérieur (2) étant constitué d'une feuille (20) comportant latéralement deux plis longitudinaux (21, 22) permettant de rabattre les parties latérales (23, 24) sur la partie centrale (25) afin de maintenir la plaquette imprégnée (3); et l'étui extérieur (1) étant constitué d'une feuille (10) pliée en deux selon deux plis longitudinaux (11, 14).

4. Dispositif selon la revendication 3 caractérisé en ce que l'étui intérieur (2) comporte un rabat solidaire du bord inférieur de la partie centrale (25), sur lequel la plaquette (3) est collée ou agrafée.

5. Dispositif selon la revendication 1 caractérisé en ce qu'un orifice (120, 130) est percé dans l'étui extérieur (1), à proximité de son bord supérieur, en vue de l'insertion d'un organe de suspension.

6. Dispositif selon la revendication 5 caractérisé en ce que l'étui intérieur (2) comporte à proximité de son bord supérieur un orifice (250) de forme oblongue dans le sens longitudinal permettant à l'orifice (120, 130) de l'étui extérieur (1) d'être en permanence en regard dudit orifice oblong (250) lors du coulissement de l'étui intérieur (2) dans l'étui extérieur (1).

## Claims

1. Deodorant perfume spray device with adjustable spraying including a first external case (1) made of a semi-rigid material comprising a certain number of openings (110, 140) into which a second internal case (2) is introduced, said case fitted with the same number of openings (26, 27) able to be placed opposite the openings (140, 110) of the external case (1) by sliding the internal case (2) into said external case (1), characterised in that the two cases (1, 2) are approximately flat and in that the openings (110, 140 ; 26, 27) are provided at the border of said cases mounted on their longitudinal folds (11, 14 ; 21, 22), the internal case (2) containing an insert (3) made of a material impregnated with a perfumed product.

2. Device according to claim 1, characterised in that the openings (110, 140) made in the external case (1) are round with a scalloping (111, 141 at the level of the fold (11, 14).

3. Device according to claim 1 or 2, characterised in that the internal case (2) and the external case (1) are made of cardboard or a plastic material, the internal case (2) being formed of a sheet (20) laterally comprising two longitudinal folds (21, 22) enabling the lateral portions (23, 24) to be folded down onto the central portion (25) so as to support the impregnated insert (3), the external case (1) being formed of a sheet (10) folded in two along two longitudinal folds (11, 14).

4. Device according to claim 3, characterised in that the internal case (2) comprises a flap integral with the lower edge of the central portion (25) on which the insert (3) is glued or stapled.

5. Device according to claim 1, characterised in that an opening (120, 130) is pierced in the external case (1) close to its upper edge with a view to inserting a suspension member.

6. Device according to claim 5, characterised in that the internal case (2) comprises close to its upper edge an oblong opening (250) in a longitudinal direction enabling the opening (120, 130) of the external case (1) to be permanently opposite said oblong opening (250) when sliding the internal case (2) into the external case (1).

## Patentansprüche

1. Diffusorvorrichtung für desodorisierendes Parfüm mit einstellbarer Diffusion, ein erste äußere Hülle (1) aus halbfestem Material umfassend, die eine bestimmte Anzahl Öffnungen (110, 140) beinhaltet, in die eine zweite, mit derselben Anzahl Öffnungen (26, 27) versehene innere Hülle (2) eingeführt wird, die dazu geeignet sind, durch Gleiten der inneren Hülle (2) in die äußere Hülle (1) gegenüber den Öffnungen (140, 110) der äußeren Hülle (1) angebracht zu werden, dadurch gekennzeichnet, daß die zwei Hüllen (1, 2) deutlich flach sind und daß die Öffnungen (110, 140; 26, 27) am Rand der besagten Hüllen rittlings auf ihren Längsfalten (11, 14; 21, 22) angebracht sind, wobei die innere Hülle (2) ein Plättchen (3) mit einem parfümierten Produkt imprägniertes Material umschließt.

2. Vorrichtung gemäß Anspruch 1, dadurch gekennzeichnet, daß die in die äußere Hülle (1) praktizierten Öffnungen (11, 140) rund sind mit einem bogenförmigen Ausschnitt (111, 141) auf der Höhe der Falten.

3. Vorrichtung gemäß Anspruch 1 oder 2, dadurch gekennzeichnet, daß die innere Hülle (2) und die äußere Hülle (1) aus Karton sind oder aus Plastik, wobei die innere Hülle (2) aus einem Blatt (20) besteht, das seitlich zwei Längsfalten (21, 22) beinhaltet, die es ermöglichen, die seitlichen Teile (23, 24) auf das zentrale Teil (25) umzuschlagen, um das imprägnierte Plättchen (3) festzuhalten; und wobei die äußere Hülle (1) aus einem gemäß zwei Längsfalten (11, 14) gefalteten Blatt (10) besteht.

4. Vorrichtung gemäß Anspruch 3, dadurch gekennzeichnet, daß die innere Hülle (2) einen mit dem unteren Rand des zentralen Teils (25) fest verbundenen Umschlag umfaßt, auf dem das Plättchen (3) festgeklebt oder festgeklemmt wird.

5. Vorrichtung gemäß Anspruch 1, dadurch gekennzeichnet, daß eine Öffnung (120, 130) in die äußere Hülle (1), in der Nähe ihres oberen Randes, gegenüber der Einfügung eines Aufhängeorgans gebohrt wird.

6. Vorrichtung gemäß Anspruch 5, dadurch gekennzeichnet, daß die innere Hülle (2) in der Nähe ihres oberen Randes eine Öffnung (250) in länglicher Form in Längsrichtung enthält, die es der Öffnung (120, 130) der äußeren Hülle (1) ermöglicht, beim Gleiten der inneren Hülle (2) in die äußere Hülle (1) ständig gegenüber der besagten länglichen Öffnung (250) zu sein.
